# EUROPEAN PATENT APPLICATION

(11) **EP 4 645 329 A1**
(43) Date of publication of application: **05.11.2025**
(21) Application number: 24836242.8
(22) Date of filing: 24.06.2024
(51) Int. Cl.: G16H 20/70, G16H 10/20, G16H 10/60, G16H 50/30, A61B 5/16, A61B 5/00, G06T 19/00, G10L 25/66

(54) **DIGITAL TREATMENT SYSTEM BASED ON COGNITIVE BEHAVIORAL THERAPY AND METHOD THEREOF**

(30) Priority: 03.07.2023 KR 20230085668
(71) Applicant: Neudive Inc., Daegu 41061 (KR)
(72) Inventor: CHO, Sung Ja, Seoul 08727 (KR)
(74) Representative: Krauns, Christian
(86) International application number: PCT/KR2024/008716
(87) International publication number: WO 2025/009794

(57) **Abstract**

A digital therapeutic system based on cognitive behavioral therapy according to an embodiment of the present invention includes a user terminal and a digital therapeutic server configured to provide cognitive behavioral therapy-based content to the user terminal, wherein the digital therapeutic server includes: a collection unit configured to collect background information of a user via the user terminal; a presentation unit configured to present at least one theme to the user terminal; a learning unit configured to provide a plurality of tasks and basic content corresponding to the theme to the user terminal when the user terminal selects one of the at least one theme; an application unit configured to provide skill enhancement content capable of applying skills corresponding to the basic content to the user terminal; a virtual field unit configured to provide virtual field content derived from the basic content or having content of the skill enhancement content reconfigured, to the user terminal; and an evaluation unit configured to evaluate content learned by the user.

## Description

### TECHNICAL FIELD

The present invention relates to a digital therapeutic system based on cognitive behavioral therapy and a method thereof. More specifically, the present invention relates to a digital therapeutic system based on cognitive behavioral therapy and a method thereof for providing personalized smart healthcare services to individuals with autism spectrum disorder aged 10 to 18 who specifically require the development of peer social situation recognition and interaction, rather than infants and toddlers.

### BACKGROUND OF THE INVENTION

Autism spectrum disorder is a mental disorder characterized by developmental deficits in social interaction, restricted interests, stereotyped behaviors, and sensory sensitivities, appearing from early life and persisting throughout life. Individuals with autism spectrum disorder exhibit developmental delays in various aspects such as cognition, language, social-emotional, physical, and behavior from the developmental process in infancy and early childhood. Developmental delays due to autism spectrum disorder affect various areas of life such as school life, occupation, social relationships, daily life, and independence, and in most cases, require assistance from surrounding people such as family, teachers, and activity assistants due to difficulties in social interaction and safety issues. Therefore, discovering and treating autism spectrum disorder has a significant impact on a child's long-term growth and development, and can have a positive impact on families and society as a whole.

However, the development of effective treatments for social interaction deficits in individuals with autism spectrum disorder is slow, and the utilization rate is low due to high costs and limited treatment facilities. Although risperidone and aripiprazole have received FDA approval for treating irritability associated with autism spectrum disorder, there are no FDA-approved standard drugs that target core symptoms such as social situation recognition and interaction problems (Research & Development Special Zone Promotion Foundation, 2021). According to the American Association of People with Disabilities, the average treatment expenditure for children with autism spectrum disorder in the US in 2017 was $17,081 per year. Furthermore, including not only direct treatment expenditures incurred by parents of children with autism spectrum disorder but also indirect costs (e.g., loss of parents' working hours, household labor costs, etc.), the average household treatment expenditure is estimated to be over $36,000 per year. In Korea, applied behavior analysis (ABA) techniques have been introduced for promoting social interaction in autism spectrum disorder for over 10 years, and treatment programs are gradually being disseminated. However, in Korea, the number of certified therapists is small and tends to be concentrated in the metropolitan area, and the utilization rate is low due to monthly medical expenses exceeding 3 million KRW, with each session costing nearly 60,000 to 100,000 KRW (Dong-A Ilbo, 2022.8.9.). Additionally, when utilizing services such as rehabilitation exercise therapy, speech therapy, art therapy, and music therapy, additional costs may be incurred, and 49.7% of parents of children with developmental disabilities responded that treatment costs are 'very burdensome' or 'somewhat burdensome' (Korea Institute of Child Care and Education, 2019).

Meanwhile, digital therapeutic devices help children learn in an engaging way, and these devices provide content in a manner that children can easily understand and find interesting by utilizing games, videos, and animation. This approach can help children enjoy learning, build confidence, and actively participate. Digital therapeutic devices are highly portable as they can be applied using smartphones, smart pads, etc., are not restricted by location, allow for multiple repetitive learnings, and are relatively inexpensive compared to face-to-face therapy. In particular, they are considered useful in that they can provide real-time feedback and solve problems when daily life issues occur.

Cognitive Behavioral Therapy (CBT) is one of the psychotherapy techniques that approaches the relationship between human thoughts, behaviors, and emotions through six stages: Assessment or psychological assessment, Reconceptualization, Skills acquisition, Skills consolidation and application training, Generalization and maintenance, and Post-treatment assessment follow-up. Cognitive behavioral therapy is known to achieve results such as cognitive improvement, behavior modification, and emotion regulation through repetitive learning, and is an evidence-based treatment method currently utilized for various mental disorders such as ADHD, mood disorders, and addiction.

Therefore, there is a growing need for a digital therapeutic system and method for individuals with autism spectrum disorder based on cognitive behavioral therapy, implemented through digital therapeutic devices.

### BRIEF SUMMARY OF THE INVENTION

### PROBLEM TO BE SOLVED

The purpose of the present invention to solve the aforementioned problems is to provide a digital therapeutic system and method based on cognitive behavioral therapy that can enhance the executive functions of individuals with autism spectrum disorder in their daily lives by using a digital therapeutic device.

### SOLUTION TO PROBLEM

To achieve the aforementioned purpose, a digital therapeutic system based on cognitive behavioral therapy according to an embodiment of the present invention may include a user terminal and a digital therapeutic server configured to provide cognitive behavioral therapy-based content to the user terminal. The digital therapeutic server may include: a collection unit configured to collect background information of a user via the user terminal; a presentation unit configured to present at least one theme to the user terminal; a learning unit configured to provide a plurality of tasks and basic content corresponding to the theme to the user terminal when the user terminal selects one of the at least one theme; an application unit configured to provide skill enhancement content capable of applying skills corresponding to the basic content to the user terminal; a virtual field unit configured to provide virtual field content derived from the basic content or having content of the skill enhancement content reconfigured, to the user terminal; and an evaluation unit configured to evaluate content learned by the user.

According to some embodiments, services provided by the collection unit, the presentation unit, the learning unit, the application unit, the virtual field unit, and the evaluation unit to the user terminal may respectively correspond to Assessment or psychological assessment, Reconceptualization, Skills acquisition, Skills consolidation and application training, Generalization and maintenance, and Post-treatment assessment follow-up of cognitive behavioral therapy.

According to some embodiments, the background information of the user collected by the collection unit includes the age and disability level of the user, and the learning unit, the application unit, and the virtual field unit are configured to adjust the difficulty level of the basic content, the skill enhancement content, and the virtual field content according to the collected age and disability level of the user.

According to some embodiments, a plurality of themes presented by the presentation unit to the user terminal may be themes according to situations that may occur in daily life.

According to some embodiments, the basic content may consist of a video including scenes corresponding to each of the plurality of tasks.

According to some embodiments, the skill enhancement content may include first skill enhancement content, wherein the first skill enhancement content is configured in a manner where a plurality of speakers converse with each other based on dialogue content included in the basic content, and one of the plurality of speakers performs a role of the user.

According to some embodiments, the skill enhancement content includes second skill enhancement content in which speech of the speaker performing the role of the user is muted, wherein the application unit is configured to provide the second skill enhancement content to the user terminal after providing the first skill enhancement content to the user terminal, wherein the user terminal is configured to record speech of the user when the muted speech of the speaker is provided, and wherein the application unit is configured to receive the recorded user's speech.

According to some embodiments, the virtual field content includes first virtual field content including a video derived from the basic content or a video with reconfigured content of the skill enhancement content, wherein the first virtual field content is configured in a manner where a plurality of speakers converse with each other, and one of the plurality of speakers performs a role of the user, wherein the virtual field content includes second virtual field content in which speech of the speaker performing the role of the user in the first virtual field content is muted, wherein the virtual field unit is configured to provide the second virtual field content to the user terminal after providing the first virtual field content to the user terminal, wherein the user terminal is configured to record speech of the user when the muted speech of the speaker is provided, and wherein the virtual field unit is configured to receive the recorded user's speech.

According to some embodiments, the evaluation unit is configured to evaluate the content learned by the user based on the user's speech respectively received by the application unit and the virtual field unit.

A digital therapeutic method based on cognitive behavioral therapy according to some embodiments of the present invention may include: collecting background information of a user via a user terminal; presenting at least one theme to the user terminal; providing a plurality of tasks and basic content corresponding to the selected theme to the user terminal when the user terminal selects one of the at least one theme; providing skill enhancement content capable of applying skills corresponding to the basic content to the user terminal; providing virtual field content derived from the basic content or having content of the skill enhancement content reconfigured, to the user terminal; and evaluating content learned by a user.

### ADVANTAGES OF THE INVENTION

According to the digital therapeutic system based on cognitive behavioral therapy and the method thereof of the present invention, by providing cognitive behavioral therapy-based educational content to individuals with autism spectrum disorder, there is an effect of obtaining results such as cognitive improvement, behavior modification, and emotion regulation through repetitive learning.

According to the digital therapeutic system based on cognitive behavioral therapy and the method thereof of the present invention, by providing educational content to individuals with autism spectrum disorder through a digital therapeutic device, there is an effect of being portable, not restricted by location, capable of multiple repetitive learnings, and relatively inexpensive compared to face-to-face therapy.

According to the digital therapeutic system based on cognitive behavioral therapy and the method thereof of the present invention, by utilizing games, videos, and animation to provide educational content to individuals with autism spectrum disorder, there is an effect of allowing individuals with autism spectrum disorder to understand educational content more easily and feel interested.

### BRIEF DESCRIPTION OF DRAWINGS

FIG. 1 is a block diagram showing each configuration of a digital therapeutic system based on cognitive behavioral therapy according to the present invention.
FIG. 2 is a block diagram showing each configuration of a digital therapeutic server according to the present invention.
FIG. 3 is a diagram illustrating an example of themes presented by a presentation unit to a user terminal.
FIG. 4 is a flowchart of a digital therapeutic method based on cognitive behavioral therapy according to the present invention.

### DETAILED DESCRIPTION OF EMBODIMENTS

Hereinafter, some embodiments of the present invention will be described in detail with reference to exemplary drawings. In attaching reference numerals to the components in each drawing, it should be noted that the same components are intended to have the same numerals as much as possible, even if they are shown in different drawings.

Furthermore, in describing embodiments of the present invention, if a detailed description of a related known configuration or function is deemed to hinder the understanding of the embodiments of the present invention, that detailed description will be omitted.

In addition, when describing components of embodiments of the present invention, terms such as first, second, A, B, (a), and (b) may be used. Such terms are merely for distinguishing the components from other components, and do not limit the nature, order, or sequence of the corresponding components.

As used herein, the singular forms include plural forms unless explicitly stated otherwise in the context. The terms "comprises" and/or "comprising" as used in the specification do not exclude the presence or addition of one or more other components in addition to the mentioned components.

Hereinafter, the present invention will be described in more detail with reference to the accompanying drawings.

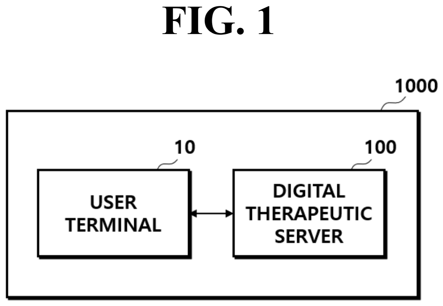

FIG. 1 is a block diagram showing each configuration of a digital therapeutic system based on cognitive behavioral therapy 1000 (hereinafter, referred to as 'system 1000') according to the present invention.

The system 1000 according to the present invention may include a user terminal 10 and a digital therapeutic server 100.

The user terminal 10 (digital therapeutic device, hereinafter, referred to as 'user terminal 10') is carried by a user with autism spectrum disorder. The user can transmit and receive information with the digital therapeutic server 100 through the user terminal 10.

An application for treating the user's autism spectrum disorder may be installed on the user terminal 10. The application is a user program for specifically performing the system 1000 and method according to the present invention. Such an application may be downloaded and installed from the digital therapeutic server 100 or another app store server. The application installed on the user terminal 10 can communicate with the digital therapeutic server 100 to receive educational content, display educational screens on the display device of the user terminal 10, vocalize audio information, or record the user's speech.

The digital therapeutic server 100 provides at least one communication protocol for communicating with the user terminal 10 via a communication network, and may provide an interface such as an application programming interface (API) for app linkage to the user terminal 10 for services related to the application.

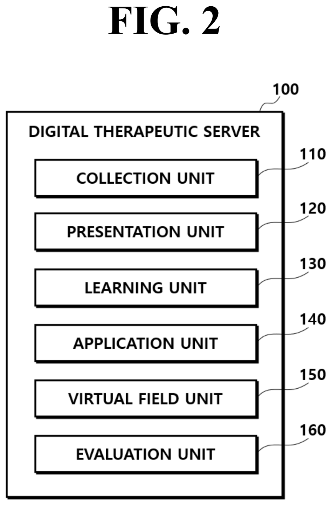

Hereinafter, with reference to FIG. 2, each configuration of the digital therapeutic server 100 according to the present invention will be described in detail.

**FIG. 2** **is** a block diagram showing each configuration of the digital therapeutic server 100.

Referring to FIG. 2, the digital therapeutic server 100 according to the present invention includes a collection unit 110, a presentation unit 120, a learning unit 130, an application unit 140, a virtual field unit 150, and an evaluation unit 160. Meanwhile, the services provided by the aforementioned collection unit 110, presentation unit 120, learning unit 130, application unit 140, virtual field unit 150, and evaluation unit 160 to the user terminal 10 may respectively correspond to ① Assessment or psychological assessment, ② Reconceptualization, ③ Skills acquisition, ④ Skills consolidation and application training, ⑤ Generalization and maintenance, and ⑥ Post-treatment assessment follow-up of cognitive behavioral therapy.

For example, the collection unit 110, corresponding to 'Assessment or psychological assessment' of cognitive behavioral therapy, identifies the user's current knowledge. The collection unit 110 can identify the user's current knowledge and collect the disability level, etc. In addition, the collection unit 110 can also collect the user's age, gender, etc.

The presentation unit 120, corresponding to 'Reconceptualization' of cognitive behavioral therapy, presents themes to be learned. Meanwhile, reconceptualization refers to the process of re-evaluating and reconstructing the way the user understands and handles the current problem situation. Therefore, the themes presented by the presentation unit can be selected based on situations that may occur in the user's daily life.

The learning unit 130, corresponding to 'Skills acquisition' of cognitive behavioral therapy, can present detailed tasks when the user selects a theme, and provide basic content including the content of those detailed tasks to the user. At this time, the basic content enables the acquisition of skills in detailed tasks through repetitive practice.

The application unit 140, corresponding to 'Skills consolidation and application training' of cognitive behavioral therapy, can provide skill enhancement content consisting solely of the speech content included in the basic content to the user. At this time, the skill enhancement content provides options for the user to select appropriate speech, or **provides** an environment where the user can actually speak, thereby enabling the application of acquired skills to real situations.

The virtual field unit 150, corresponding to 'Generalization and maintenance' of cognitive behavioral therapy, can provide virtual field content to the user, allowing for repetitive practice of tasks that can be derived from the basic content and skill enhancement content, or situations that may additionally occur for each theme, in a virtual field. According to an embodiment, the virtual field content provides options for the user to select appropriate speech in a virtual field related to the theme presented by the presentation unit 120, or provides an environment where the user can actually speak, allowing the user to apply acquired skills in the virtual field.

The evaluation unit 160, corresponding to 'Post-treatment assessment follow-up' of cognitive behavioral therapy, can evaluate and repeat the overall process. For example, the evaluation by the evaluation unit 160 can evaluate the user's education and learning results based on the options selected by the user and/or the actual speech made by the user during the skill enhancement content of the application unit 140 and/or the virtual field content of the virtual field unit 150.

Cognitive behavioral therapy, as described above, is one of the psychotherapy techniques that approaches the relationship between human thoughts, behaviors, and emotions within a process encompassing ① Assessment or psychological assessment, ② Reconceptualization, ③ Skills acquisition, ④ Skills consolidation and application training, ⑤ Generalization and maintenance, and ⑥ Post-treatment assessment follow-up. Cognitive behavioral therapy is known to achieve results such as cognitive improvement, behavior modification, and emotion regulation through repetitive learning, and is an evidence-based treatment method currently utilized for various mental disorders such as ADHD, mood disorders, and addiction.

Meanwhile, adolescents and adults with autism spectrum disorder often experience significant problems with daily life adaptation due to executive function issues, even when intellectual disability is not present. In particular, they often have difficulty completing target behaviors due to problems with key executive functions such as **Cognitive** flexibility, Self-control (of emotions and impulses), Working memory, and Planning and Initiation.

Studies have been published that identified individual executive function vulnerabilities in individuals with autism spectrum disorder and demonstrated effectiveness by providing customized programs for executive function enhancement (Chung, 2016; Oswald, 2017; Yang, 2022; de Vries, 2015; Kenworthy, 2013; Baker-Ericzén, 2017). These studies demonstrated improvements in working memory, behavioral control, planning, and organizational skills among executive functions, and also proved effective in reducing anxiety.

However, these studies were mostly conducted under controlled experimental conditions, reporting only limited indirect effects in the process of increasing daily life adaptability and social interaction, thus having limitations in claiming to have performed real-world based social behavior enhancement.

Therefore, the system 1000 according to the present invention can educate and treat individuals with autism spectrum disorder in daily life adaptation and social interaction by: ① the collection unit 110 identifying the user's current knowledge (background information) via the user terminal 10; ② the presentation unit 120 presenting at least one theme to the user terminal 10 and receiving a selection of one of the at least one theme from the user terminal 10; ③ the learning unit 130 providing a plurality of detailed tasks corresponding to the theme along with repetitive practice-capable basic content to the user terminal 10 to enable skill acquisition when the user terminal 10 selects one of the at least one theme; ④ the application unit 140 providing skill enhancement content to the user terminal to enable application of skills acquired through the basic content; ⑤ the virtual field unit 150 providing virtual field content, which includes tasks derivable from the basic content or reconfigured content of the skill enhancement content, to facilitate repetitive practice in ever-changing real-world situations; and ⑥ the evaluation unit 160 evaluating and learning the learned content.

The collection unit 110 can collect background information of a user with autism spectrum disorder (ASD) via the user terminal 10 to identify the user's current knowledge. **For example,** the collection unit 110 collects the user's age, disability level, gender, and so on.

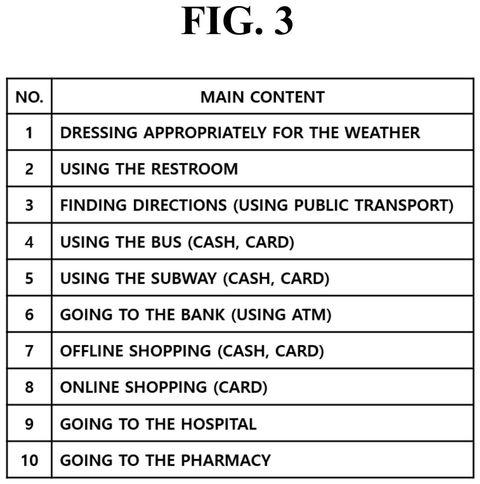

FIG. 3 is a diagram illustrating an example of themes presented by the presentation unit 120 to the user terminal 10.

The presentation unit 120 can present at least one theme to the user terminal 10. The themes presented by the presentation unit 120 can be configured to allow practice of communication processes with individuals encountered in daily or social situations, considering the deficits in social interaction and communication abilities of individuals with autism spectrum disorder. This corresponds to the objective of the present invention to provide personalized smart healthcare services to individuals with autism spectrum disorder aged 10 to 18 who require focused development of peer social situation recognition and interaction, rather than infants and toddlers. As the themes presented by the presentation unit 120 involve situations that may occur in daily life, the basic content provided by the learning unit 130 (to be described later) also includes content about communication processes with individuals encountered in daily or social situations, thereby comprehensively promoting planning and organization, cognitive flexibility, and social interaction in the process of adapting to daily life for individuals with autism spectrum disorder.

As described above, the themes presented by the presentation unit 120 to the user terminal 10 may be themes that can occur in daily life, and for example, as shown in FIG. 3, they include: ① dressing appropriately for the weather, ② using the restroom, ③ finding directions (using public transport), ④ using the bus (cash, card), ⑤ using the subway (cash, card), ⑥ going to the bank (using ATM), ⑦ offline shopping (cash, card), ⑧ online shopping (card), ⑨ going to the hospital, and ⑩ going to the pharmacy.

When the presentation unit 120 presents the aforementioned themes to the user terminal 10, the user terminal 10 can select one of the themes presented by the presentation unit 120. Meanwhile, as an embodiment, a guardian of an individual with autism spectrum disorder, for example, a parent, may select one of the aforementioned themes. Although not shown in the drawing, if a guardian intervenes in the service **provided** by the present invention, the guardian can connect to the digital therapeutic server 100 through a guardian terminal (not shown).

When the user terminal 10 selects one of the aforementioned themes, the presentation unit 120 receives information about the selection and transmits that information to the learning unit 130.

The learning unit 130 can provide a plurality of detailed tasks corresponding to that theme to the user terminal 10.

As an embodiment, if the theme 'dressing appropriately for the weather' is selected, the learning unit 130 presents detailed tasks such as: ① checking the weather, ② checking the temperature, ③ deciding the general category of clothes, ④ choosing appropriate clothes from what I have, and ⑤ identifying inappropriate clothes.

Alternatively, as an embodiment, if the theme 'using the bus (card)' is selected, the learning unit 130 presents detailed tasks such as: ① searching for departure and destination via Naver Map or Kakao Map application, ② checking travel time, ③ preparing to depart 20 minutes before the travel time, ④ checking the bus number to take, ⑤ checking the bus stop, ⑥ waiting for the bus at the bus stop, ⑦ holding the transit card in hand beforehand, ⑧ when the bus arrives, boarding and tapping the transit card (e.g., scene of dragging and tapping the transit card), ⑨ taking a seat, ⑩ checking the stop to get off at, ⑪ listening carefully to the bus announcement and pressing the bell, and ⑫ tapping the card and getting off.

Alternatively, as an embodiment, if the theme 'using the subway (card)' is selected, the learning unit 130 presents detailed tasks such as: ① searching for departure and destination via Naver Map or Kakao Map application, ② checking the number of subway transfers, ③ checking the subway station, ④ checking travel time, ⑤ preparing to depart 20 minutes before the travel time, ⑥ checking the name of the station to get off at, ⑦ listening carefully to the subway announcement and preparing to get off, ⑧ after getting off, checking the map to find which exit number to use, ⑨ when the announcement is made, getting off and looking for the ticket gate by referring to the exit **sign,** ⑩ tapping the transit card to exit, and ⑪ finding the exit by referring to the exit sign.

Alternatively, as an embodiment, if the theme 'going to the bank (using ATM - withdrawal)' is selected, the learning unit 130 presents detailed tasks such as: ① checking the bank mark on the check card (e.g., bank names appear by bank mark), ② searching for the bank name on Naver Map or Kakao Map, ③ going to the nearest bank, ④ opening the door and entering where the ATM is located, ⑤ clicking the deposit/withdrawal button, ⑥ clicking "Card", ⑦ inserting the card into the flashing arrow direction, ⑧ selecting the amount, ⑨ if the amount is not listed, selecting "Other" then entering the number and pressing the confirm button, ⑩ confirming the amount and pressing confirm, ⑪ entering the 4-digit card PIN, ⑫ printing the statement, and ⑬ receiving the card and statement.

Alternatively, as an embodiment, if the theme 'online shopping (card - app card installation guide)' is selected, the learning unit 130 presents detailed tasks such as: ① searching for "mask" on Naver, ② tapping the shopping tab, ③ viewing desired color and size and clicking, ④ after selecting color and size, confirming the quantity to purchase, ⑤ tapping "Purchase", ⑥ searching and entering home address for delivery, ⑦ entering mobile phone number, ⑧ entering delivery message, ⑨ tapping "General Payment", ⑩ selecting "Credit Card", ⑪ clicking the company name of the card I have, ⑫ tapping "Pay", ⑬ tapping "App Card Payment", ⑭ tapping QR code to pay, and ⑮ entering simple payment password.

Meanwhile, each of the tasks presented by the learning unit 130 can consist of a single scene in the basic content.

The learning unit 130 can provide basic content according to the user's selected theme and the plurality of tasks corresponding to that theme to the user terminal 10. The basic content provided by the learning unit 130 to the user terminal 10 consists of a video including scenes corresponding to the tasks of the selected theme. The basic content can, for example, consist of an animation in which multiple speakers converse with each other. The video of the basic content may include not only the conversations of multiple speakers but also separate compositions for explaining each theme and task, and for helping the user understand the current situation.

Meanwhile, the learning unit 130 can adjust the difficulty level of the basic content according to the user's background information collected by the collection unit 110. For example, the learning unit 130 adjusts the difficulty level of the basic content according to the user's age and disability level. At this time, the difficulty level of the basic content is adjusted by controlling the number of speakers or the number of tasks.

As the number of speakers increases, the amount of conversation exchanged also increases, and a higher level of conversational ability is required. For example, when there are 2 speakers, they can take turns speaking and only focus their gaze on the other person, but when there are 4 speakers, they must remember what the 3 other people are saying and respond in accordance with the flow of conversation, and the timing of speech and gaze processing become more complex.

Also, if the number of tasks increases, the difficulty level decreases, and if the number of tasks decreases, the difficulty level increases. For example, in the case of the task 'finding the exit after getting on the subway', if it is divided into detailed tasks such as 'check the station to get off at > listen carefully to the announcement > check the exit on the map', the difficulty level for each task decreases.

Meanwhile, this difficulty adjustment is not only applied to the basic content of the learning unit 130 but can similarly be applied to the skill enhancement content of the application unit 140 and/or the virtual field content of the virtual field unit 150, which will be described later.

The application unit 140 can provide skill enhancement content to the user terminal 10 that allows for applying and utilizing skills corresponding to the basic content. The skill enhancement content can primarily consist of a method where a plurality of speakers converse with each other, focusing solely on conversation training based on the dialogue content included in the basic content. At this time, one of the speakers performs the role of the user. When the speaker performing the user's role speaks, the animation within the skill enhancement content is partially modified to indicate that speaker, so that attention can be focused on that speech. Regarding the speaker's indication, the application unit 140 can implement real-time changing 2D or 3D model facial and/or expression animation by assigning feature points to the user's face region captured by the user terminal 10's camera, and calculating and digitizing the amount of change in the assigned feature points using interpolation, which will be described later.

When providing the skill enhancement content to the user terminal 10, the application unit 140 can provide the skill enhancement content after muting the speech of the speaker who performed the user's role in the skill enhancement content. That is, the application unit 140 can provide the aforementioned general skill enhancement content (e.g., 'first skill enhancement content') to the user terminal 10 for a preset number of times determined according to the user's age and disability level, and then provide skill enhancement content (e.g., 'second skill enhancement content') in which only the speech of the speaker who performed the user's role is muted, to the user terminal 10. Through the second skill enhancement content, the application unit 140 presents a video in which the speech of the character representing the user is omitted during video playback, and allows the user to learn verbal communication expressions by completing the omitted dialogue.

When the speech of the character representing the user is omitted in the second skill enhancement content, the application unit 140 can display a plurality of options on the user terminal 10 for selecting appropriate speech content for that character. The user can select the option with the appropriate speech from the displayed options.

Alternatively, when the speech of the character representing the user is omitted in the second skill enhancement content, the application unit 140 can provide an environment where the user can actually speak the appropriate speech content for that character. The user terminal 10 can record the user's speech when the muted speech of the speaker is provided during the playback of the second skill enhancement content. The user terminal 10 transmits the selected option information and/or the recorded user's speech to the application unit 140, and the application unit 140 transmits the received option information and/or the recorded user's speech to the evaluation unit 160. The evaluation unit 160 can evaluate the user's learning content based on the user's selected option information and/or recorded speech according to criteria to be described later.

The virtual field unit 150 can provide virtual field content to the user terminal 10, which includes tasks derivable from the theme and/or basic content, or reconfigured speech of the skill enhancement content.

The virtual field content provides options for the user to select appropriate speech in a virtual field related to the theme presented by the presentation unit 120, or provides an environment where the user can actually speak, allowing the user to apply acquired skills in the virtual field.

The virtual field content is intended for learning and educating how to appropriately respond to various changing situations, considering the characteristic of individuals with autism spectrum disorder who often find it difficult to flexibly adapt and apply in response to various changes.

For example, if the theme selected by the user is 'dressing appropriately for the weather', the detailed tasks corresponding to it, as described above, are: ① checking the weather, ② checking the temperature, ③ deciding the general category of clothes, ④ choosing appropriate clothes from what I have, and ⑤ identifying inappropriate clothes. The basic content is a video containing scenes corresponding to each detailed task, and the skill enhancement content is constructed by extracting only the speech content from each basic content. At this time, the virtual field content includes, for example, content that can guide methods such as borrowing an umbrella or purchasing an inexpensive umbrella in situations where the weather forecast differs from the actual weather, or when it suddenly rains, which can be derived from the aforementioned themes and tasks. Additionally, the virtual field content may be a reconfiguration of the speech content of the skill enhancement content, which was constructed by extracting only the speech content from each basic content. Reconfiguration at this time may mean changing the speaker from female to male, changing the speaker's age group, or changing the number of speakers. Therefore, the virtual field content presents various changing situations based on themes and detailed tasks, and enables education and learning of appropriate speech accordingly.

The virtual field content, like the aforementioned skill enhancement content, includes a speaker who performs the role of the user, and includes a first virtual field content that completely includes the content of that speech, and a second virtual field content in which the speech of the speaker performing the role of the user is muted during their speech.

That is, the first virtual field content includes a video containing content derived from the basic content and/or a video with reconfigured content of the skill enhancement content. The first virtual field content is configured in a manner where a plurality of speakers converse with each other, and one of the speakers performs the role of the user.

The second virtual field content, in which the speech of the speaker performing the role of the user in the first virtual field content is muted, can be provided to the user.

The virtual field unit 150 can provide the first virtual field content to the user terminal 10 for a preset number of times determined according to the user's age and disability level, and then provide the second virtual field content, in which only the speech of the speaker who performed the user's role is muted, to the user terminal 10. Through the second virtual field content, the virtual field unit 150 presents a video in which the speech of the character representing the user is omitted during video playback, and allows the user to learn verbal communication expressions by applying the speech learned in the basic content and skill enhancement content to complete the omitted dialogue.

At this time, the virtual field unit 150 can receive option information and/or speech information selected or recorded through the virtual field content from the user terminal 10, and the virtual field unit transmits the received option information and/or recorded speech information to the evaluation unit 160. The evaluation unit 160 can evaluate the content learned by the user based on the user's selected option information and/or recorded speech according to criteria to be described later.

The evaluation unit 160 can evaluate the content learned by the user based on the correctness of the option information and/or the recorded user's speech respectively received by the application unit 140 and/or the virtual field unit 150.

If the evaluation unit 160 evaluates the content learned by the user based on the recorded user's speech, the evaluation unit 160 evaluates the user's learned content based on a plurality of evaluation criteria. At this time, the evaluation criteria include a keyword evaluation criterion, a conversation response speed evaluation criterion, a speaking speed evaluation criterion, and a conversation repetition evaluation criterion.

The evaluation unit 160 analyzes and evaluates the audio of the recorded user's speech content, then determines learning training as successful if the user's conversation content meets preset evaluation criteria, and may determine learning training as failed if the user's conversation content does not meet the evaluation criteria.

The keyword evaluation criterion is for identifying and evaluating whether keywords are included in the user's speech.

The conversation response speed evaluation criterion is an evaluation of how appropriately fast the user spoke after the other person finished speaking. If the time to listen to the other person's words and respond is long, it hinders smooth conversation, such as causing conversation to stop or being rushed. Moreover, if the user speaks before the other person finishes speaking, the conversation can be interrupted, or the user may have spoken entirely different words unrelated to the other person's statement.

The speaking speed evaluation criterion is for evaluating the speaking speed during a conversation. Speaking speed is an important skill for effectively conveying words to the other person. If it is excessively fast or slow, it can be difficult for the other person to understand what the speaker is saying or to pay attention to the speaker's words, making it difficult to maintain a conversation.

The conversation repetition evaluation criterion evaluates whether the user repeats the same words or phrases when speaking.

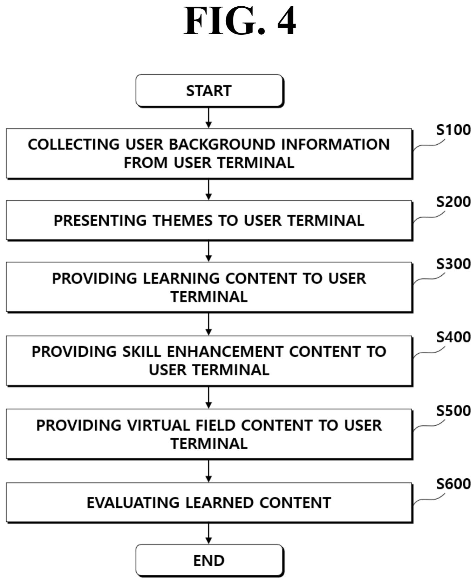

FIG. 4 is a flowchart of a digital therapeutic method based on cognitive behavioral therapy according to the present invention.

The digital therapeutic method based on cognitive behavioral therapy (hereinafter, referred to as 'method') according to the present invention, referring to FIG. 4, may include: collecting background information of a user from the user terminal 10 by the collection unit 110 (S100); presenting at least one theme to the user terminal 10 by the presentation unit 120 (S200); when the user terminal selects one of the themes, providing a plurality of tasks and basic content corresponding to the selected theme to the user terminal by the learning unit 130 (S300); providing skill enhancement content capable of applying skills corresponding to the basic content to the user terminal 10 by the application unit 140 (S400); providing virtual field content derived from the basic content or having content of the skill enhancement content reconfigured, to the user terminal 10 by the virtual field unit 150 (S500); and evaluating content learned by a user by the evaluation unit 160 (S600). Detailed matters for each step are as described above.

Meanwhile, the method may further include a step (not shown) of providing a reward to the user after step S600.

The step of providing a reward to the user can provide a first reward to the user terminal 10 when the evaluation by the evaluation unit 160 is completed.

At this time, the step of providing a reward to the user can provide a first reward along with a second reward to the user terminal 10 when the user's conversation content is determined to be a successful learning training by the evaluation unit 160, meeting preset evaluation criteria.

For this purpose, the system 1000 according to the present invention may further include a reward unit (not shown).

The reward unit provides a first reward to the user terminal 10 upon receiving evaluation result information from the evaluation unit 160. At this time, the first reward is given regardless of the outcome, once the user completes learning a theme and its corresponding tasks. Therefore, the magnitude of the first reward may be smaller than other rewards to be described later.

On the other hand, the second reward is given when the user achieves a successful learning training by producing results that meet the evaluation criteria for a theme and its corresponding tasks, so it may be a greater reward than the first reward. The reward unit that provided the second reward to the user transmits the result to the collection unit 110, the presentation unit 120, and/or the learning unit 130. If the user receives the second reward, the collection unit 110 collects and stores this as part of the user's background information. If the user receives the second reward, the presentation unit 120 may display a 'learning training successful' mark on that theme for a certain period, or may not present that theme to the user terminal 10 for a certain period. If the user reselects the theme for which they received the second reward, the learning unit 130 can increase the difficulty level of the basic content for that theme. Therefore, as described above, the learning unit can provide basic content with an increased number of speakers or a reduced number of tasks to the user terminal 10. Meanwhile, as repetitive learning of the same theme and tasks is important for individuals with autism spectrum disorder, if the user repeatedly learns the basic content for the theme and tasks for which they received the second reward (with increased difficulty) and the evaluation unit 160 again evaluates it as a successful learning training, the evaluation unit 160 provides a third reward to the user. Thus, the reward unit not only encourages participation in learning by providing rewards for repetitive learning but also motivates voluntary repeated participation in themes and tasks learned within a certain period.

Meanwhile, the aforementioned first to third rewards may also be provided by the guardian terminal. For example, each reward can be applied as mileage or points that can be exchanged for physical rewards. Alternatively, each reward may take the form of a symbolic icon that can be directly exchanged for a physical reward. For example, the symbolic icon at this time may refer to physical items that the user likes, such as chocolate, strawberries, or snacks, and the guardian terminal can directly provide that reward to the user through the icon received by the user. This allows for providing substantial rewards to the user, thereby motivating learning participation and repeated participation.

Also, for example, if each reward is mileage or points, the reward unit can categorize the themes learned by the user and determine highly similar themes among them as similar themes.

The reward unit can separately calculate the reward amount based on the difficulty level and average time spent on similar themes. The reward amount calculated in this way is determined as an estimated reward amount and can be displayed on the user terminal 10 along with the theme when the user selects it.

This is to provide a larger reward to the user when repeatedly learning similar themes. Furthermore, learning similar themes has the characteristic of increasing learning efficiency.

Meanwhile, as described above, the application unit 140 and the virtual field unit 150 respectively provide skill enhancement content and virtual field content to the user terminal 10. In addition, there is a speaker who performs the role of the user in the skill enhancement content and virtual field content. At this time, the application unit 140 and/or the virtual field unit 150 can recognize the user's face or expression and provide an animation to the user terminal 10 that synthesizes the user's current face or expression onto the face of the speaker performing the user's role in real-time.

The user terminal 10 captures the face of the user who is learning through the camera and transmits the captured face to the digital therapeutic system 1000. The application unit 140 and/or the virtual field unit 150 can synthesize the received user's face or expression onto the face of the speaker performing the user's role in the animation.

Accordingly, the user can recognize the speaker, onto whom their own current face or expression is synthesized, as the speaker performing their role, and by itself, their gaze is drawn to that speaker, thereby improving learning efficiency. This not only provides fun in learning to the user but also allows for real-time confirmation that the user is watching the skill enhancement content and/or virtual field content within the correct area.

The application unit 140 and the virtual field unit 150 can implement real-time changing 2D or 3D model facial and/or expression animation by assigning feature points to the face region captured by the user terminal 10's camera, and calculating and digitizing the amount of change in the assigned feature points using interpolation.

The application unit 140 and the virtual field unit 150 can each search for the user's face region in an image captured by the user terminal 10's camera, assign feature points to the detected face region, digitize the amount of change in the feature points when input, and apply the digitized amount of change to a preset 2D or 3D model with feature points and control points according to the amount of change, thereby implementing real-time changing 2D or 3D model facial and/or expression animation. When applying the digitized amount of change, the application unit 140 and the virtual field unit 150 each load a preset 2D or 3D model with feature points and control points, and if a change in the digitized feature points is recognized, control the change in feature points by the preset control points and output it, and if no change in feature points is transmitted, output the initialized face and/or expression of the loaded model. At this time, the digitized amount of change is preferably a vector function value with respect to time.

Hereinafter, a more specific explanation will be given regarding the real-time synthesis and implementation of the user's current face or expression onto the face of the speaker performing the user's role by the application unit 140 and the virtual field unit 150.

The application unit 140 and the virtual field unit 150 each capture the face and/or expression through a camera to detect the face region, and assign feature points to the eyes, nose, mouth, eyebrows, and overall facial contour within the detected face region. Then, by digitizing the amount of change in the feature points that changes as the face and/or expression changes and applying this to a preset 2D or 3D model with feature points and control points according to the amount of change in the feature points, real-time changing 2D or 3D model facial and/or expression animation can be implemented.

When applying the digitized amount of change in feature points, a preset 2D or 3D model with feature points and control points that will change according to the feature points and the amount of change in feature points is loaded, and it is checked whether the digitized amount of change in feature points is received. If received, the amount of change in feature points is controlled by the preset control points and output. If the amount of change in feature points is not received, the initialized face and/or expression animation of the loaded model is output. Therefore, it is possible to recognize when the user is not watching the skill enhancement content and virtual field content or is watching outside a certain area.

Meanwhile, the amount of change in feature points within the face region captured by the camera can be calculated through interpolation.

Interpolation is a technique for generating facial expression animation by approximating intermediate steps from faces and/or expressions represented by a plurality of feature points. There are various known methods such as Shape Interpolation, which approximates geometric structures, and Key Frame Interpolation, which approximates images over time.

Hereinafter, an embodiment of the process to which interpolation is applied will be described in detail.

First, still images are extracted from real-time incoming moving images at regular time intervals, and feature points are assigned within the detected face region in the still images and temporarily stored in a first data memory. Then, after a certain period (e.g., 0.1 seconds to 1 second) has elapsed, still images are extracted from the incoming moving images, feature points are assigned within the detected face region, and temporarily stored in a second data memory. Subsequently, the feature points temporarily stored in the first data memory and the second data memory are compared to calculate and digitize their amount of change. At this time, the amount of change is approximated as a vector function value with respect to time. Then, facial expression animation can be implemented by loading a 2D or 3D model with preset feature points and applying the digitized amount of change in feature points.

The user terminal 10 and the guardian terminal according to the present invention are terminals such as desktop PCs, laptop PCs, tablet PCs, smartphones, etc., equipped with input means such as a keyboard, mouse, touchpad, touch screen, and a display screen, but are not limited thereto. Any configuration capable of processing digital information that can connect to the digital therapeutic server 100 via a communication network, and install application programs capable of inputting search information and selection information, and displaying searched result information, can be included. The user terminal 10 according to the present invention is a component that connects to the digital therapeutic server 100 via a communication network to transmit and receive information, and for example, may include at least one of a smartphone, tablet personal-computer, mobile phone, video phone, desktop-personal computer, laptop personal computer, netbook computer, PDA (personal digital assistant), PMP (portable multimedia player), wearable device (e.g., smart glasses, head-mounted-device (HMD) etc.), kiosk, or smart watch.

The user terminal 10 and the digital therapeutic server 100 according to the present invention can communicate through their respective communication units and a communication network. A communication network refers to a connection structure that allows information exchange between individual nodes such as terminals and servers, and examples of such communication networks include, but are not limited to, 3GPP (3rd Generation Partnership Project) network, LTE (Long Term Evolution) network, 5G network, WIMAX (World Interoperability for Microwave Access) network, Internet, LAN (Local Area Network), Wireless LAN (Wireless Local Area Network), WAN (Wide Area Network), PAN (Personal Area Network), wifi network, Bluetooth network, satellite broadcasting network, analog broadcasting network, and DMB (Digital Multimedia Broadcasting) network. The communication units equipped in the user terminal 10 and the digital therapeutic server 100, respectively, may include electronic components provided for the communication network to perform wired and wireless data communication through the aforementioned communication network.

In this specification, 'unit' includes a unit realized by hardware, a unit realized by software, and a unit realized by using both. Also, one unit may be realized by using two or more hardware components, and two or more units may be realized by one hardware component.

The scope of protection of the present invention is not limited by the description and expression of the embodiments explicitly described above. Furthermore, it is once again added that the scope of protection of the present invention cannot be limited by obvious changes or substitutions within the technical field to which the present invention belongs.

## Claims

1. A digital therapeutic system based on cognitive behavioral therapy, comprising: a user terminal; and a digital therapeutic server configured to provide cognitive behavioral therapy-based learning content to the user terminal,
wherein the digital therapeutic server comprises:
a collection unit configured to collect background information of a user via the user terminal;
a presentation unit configured to present at least one theme to the user terminal;
a learning unit configured to provide a plurality of tasks and basic content corresponding to the theme to the user terminal when the user terminal selects one of the at least one theme;
an application unit configured to provide skill enhancement content capable of applying skills corresponding to the basic content to the user terminal;
a virtual field unit configured to provide virtual field content derived from the basic content or having content of the skill enhancement content reconfigured, to the user terminal; and
an evaluation unit configured to evaluate content learned by the user.

2. The digital therapeutic system based on cognitive behavioral therapy of claim 1, wherein services provided by the collection unit, the presentation unit, the learning unit, the application unit, the virtual field unit, and the evaluation unit to the user terminal respectively correspond to Assessment or psychological assessment, Reconceptualization, Skills acquisition, Skills consolidation and application training, Generalization and maintenance, and Post-treatment assessment follow-up of cognitive behavioral therapy.

3. The digital therapeutic system based on cognitive behavioral therapy of claim 1, wherein the background information of the user collected by the collection unit includes the age and disability level of the user, and the learning unit, the application unit,
and the virtual field unit are configured to adjust the difficulty level of the basic content, the skill enhancement content, and the virtual field content according to the collected age and disability level of the user.

4. The digital therapeutic system based on cognitive behavioral therapy of claim 1, wherein a plurality of themes presented by the presentation unit to the user terminal are themes according to situations that may occur in daily life.

5. The digital therapeutic system based on cognitive behavioral therapy of claim 4, wherein the basic content consists of a video including scenes corresponding to each of the plurality of tasks.

6. The digital therapeutic system based on cognitive behavioral therapy of claim 5, wherein the skill enhancement content includes first skill enhancement content, wherein the first skill enhancement content is configured in a manner where a plurality of speakers converse with each other based on dialogue content included in the basic content, and one of the plurality of speakers performs a role of the user.

7. The digital therapeutic system based on cognitive behavioral therapy of claim 6, wherein the skill enhancement content includes second skill enhancement content in which speech of the speaker performing the role of the user is muted,
wherein the application unit is configured to provide the second skill enhancement content to the user terminal after providing the first skill enhancement content to the user terminal,
wherein the user terminal is configured to record speech of the user when the muted speech of the speaker is provided, and
wherein the application unit is configured to receive the recorded user's speech.

8. The digital therapeutic system based on cognitive behavioral therapy of claim 7, wherein the virtual field content includes first virtual field content including a video derived from the basic content or a video with reconfigured content of the skill enhancement content,
wherein the first virtual field content is configured in a manner where a plurality of speakers converse with each other, and one of the plurality of speakers performs a role of the user,
wherein the virtual field content includes second virtual field content in which speech of the speaker performing the role of the user in the first virtual field content is muted,
wherein the virtual field unit is configured to provide the second virtual field content to the user terminal after providing the first virtual field content to the user terminal,
wherein the user terminal is configured to record speech of the user when the muted speech of the speaker is provided, and
wherein the virtual field unit is configured to receive the recorded user's speech.

9. The digital therapeutic system based on cognitive behavioral therapy of claim 8, wherein the evaluation unit is configured to evaluate the content learned by the user based on the user's speech respectively received by the application unit and the virtual field unit.

10. A digital therapeutic method based on cognitive behavioral therapy, comprising:
collecting background information of a user via a user terminal; presenting at least one theme to the user terminal;
providing a plurality of tasks and basic content corresponding to the selected theme to the user terminal when the user terminal selects one of the at least one theme;
providing skill enhancement content capable of applying skills corresponding to the basic content to the user terminal;
providing virtual field content derived from the basic content or having content of the skill enhancement content reconfigured, to the user terminal; and
evaluating content learned by a user.
